(19) Europäisches Patentamt
European Patent Office
Office européen des brevets

(11) **EP 4 215 226 A1**

(12) **EUROPEAN PATENT APPLICATION**

(43) Date of publication:
**26.07.2023 Bulletin 2023/30**

(21) Application number: **23151656.8**

(22) Date of filing: **16.01.2023**

(51) International Patent Classification (IPC):
***A61M 1/36*** (2006.01)

(52) Cooperative Patent Classification (CPC):
**A61M 1/3643; A61M 1/3693;** A61M 2205/3334

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **19.01.2022 US 202263300895 P**

(71) Applicant: **Fenwal, Inc.**
**Lake Zurich, IL 60047 (US)**

(72) Inventor: **Kusters, Benjamin E.**
**Lake Zurich, 60047 (US)**

(74) Representative: **Maikowski & Ninnemann**
**Patentanwälte Partnerschaft mbB**
**Postfach 15 09 20**
**10671 Berlin (DE)**

(54) **DEVICES AND METHODS FOR PROCESSING WHOLE BLOOD USING FLOW RATE STOPPAGE PHASE**

(57) A device and method for separating whole blood includes flowing whole blood to a centrifuge, separating whole blood into blood components within the centrifuge, and flowing separated blood components out of the centrifuge. The device and method include a flow rate stoppage phase executed one or more times during the method. The flow rate stoppage phase includes (i) stopping the flow of whole blood to the centrifuge and stopping the flow of separated blood components out of the centrifuge; (ii) spinning the centrifuge at a selected rate; and (iii) after a selected time ending the flow rate stoppage phase and resuming the flow of whole blood to the centrifuge and the flow of separated blood components out of the centrifuge.

FIG. 5B

Processed by Luminess, 75001 PARIS (FR)

**Description**

TECHNICAL FIELD

The present disclosure relates generally to devices and methods for processing whole blood, and more particularly to devices and methods of separating whole blood into red blood cell and plasma products.

BACKGROUND

It is well known to collect whole blood from donors using manual collection procedures through blood drives, donor visits to blood centers or hospitals and the like. In such procedures, blood is typically collected by simply flowing it from the donor under the force of gravity and venous pressure into a collection container (e.g., a flexible pouch or bag). Although various blood collection instruments may be used to aid or expedite the collection of blood or blood components.

The collection container in manual collection is often part of a larger pre-assembled arrangement of tubing and containers (sometimes called satellite containers) that are used in further processing of the collected whole blood. More specifically, the whole blood is typically first collected in what is called a primary collection container that also contains an anticoagulant, such as but not limited to a solution of sodium citrate, phosphate, and dextrose ("CPD").

After initial collection, it is a common practice to transport the collected whole blood to another facility or location, sometimes called a "back lab," for further processing to separate red blood cells, platelet, and plasma from the whole blood, which may include carrying out additional processes, such as cell washing and plasma cryoprecipitate production and collection. This processing usually entails manually loading the primary collection container and associated tubing and satellite containers into a centrifuge to separate the whole blood into concentrated red cells and platelet-rich or platelet-poor plasma. The separated components may then be expressed from the primary collection container into one or more of the satellite containers, with the red blood cells being combined with an additive or preservative solution pre-filled in one of the satellite containers. After the above steps, the blood components may be again centrifuged, if desired, for example to separate platelets from plasma. The overall process requires multiple large floor centrifuges and fluid expression devices. Because of the multiple operator interactions, the process is labor intensive, time consuming, and subject to human error.

Thus, there have been continuing efforts to automate the apparatus and systems used in the post-collection processing of whole blood, and recently it has been proposed to employ an automated blood component separator for such post-collection processing. The subject matter disclosed herein provides further advances in various aspects of the apparatus, systems and methods that may be employed in whole blood collection and post-collection processing systems by using continuous flow centrifugation in a system that utilizes a programmable controller that is pre-programed to automatically perform selected back lab processes and may also be programmed by the user to meet needs and requirements specific to the user.

SUMMARY

There are several aspects of the present subject matter which may be embodied separately or together in the devices, systems, and methods described and/or claimed below. These aspects may be employed alone or in combination with other aspects of the subject matter described herein, and the description of these aspects together is not intended to preclude the use of these aspects separately or the claiming of such aspects separately or in different combinations as set forth in the claims appended hereto or later amended. For purposes of this description and claims, unless otherwise expressly indicated, "blood" is intended to include whole blood and blood components, such as concentrated red cells, plasma, platelets and white cells, whether with or without anticoagulant or additives.

The following summary is to acquaint the reader generally with various potential aspects of the present subject matter, and is non-limiting and non-exclusive with respect to the various possible aspects or combinations of aspects. Additional aspects and features may be found in the detailed description herein and/or in the accompanying figures.

By way of the present disclosure, a method is provided for separating whole blood including executing a priming stage in which a pump system and a valve system of a blood processing device are controlled to prime a processing chamber positioned within a centrifuge of the blood processing device. A blood separation stage is executed in which the pump system, the valve system, and the centrifuge are controlled to separate blood in the processing chamber into at least two blood components. A blood component collection stage is executed in which the pump system and the valve system are controlled to collect at least a portion of one of said at least two blood components. A flow rate stoppage phase is executed to interrupt at least one of the priming, blood separation, and blood component collection stages, the flow rate stoppage phase including: (i) controlling the pumping system and the valve system to prevent fluid flow into and from the processing chamber, (ii) controlling the centrifuge at a selected rate and/or a selected relative centrifugal force; (iii) after a selected time, ending the flow rate stoppage phase; and (iv) resuming the interrupted stage or advancing to a subsequent stage of the method after ending the flow rate stoppage phase.

In another aspect, a blood processing device includes a pump system; a valve system; a centrifuge; and a controller. The controller is configured to execute a blood separation procedure including: executing a priming stage in which the pump system and the valve system are controlled to prime a processing chamber positioned within the centrifuge; executing a blood separation stage in which the pump system, the valve system, and the centrifuge are controlled to separate blood in the processing chamber into at least two blood components; executing a blood component collection stage in which the pump system and the valve system are controlled to collect at least a portion of one of said at least two blood components; and executing a flow rate stoppage phase to interrupt at least one of the priming, blood separation, and blood component collection stages. The flow rate stoppage phase includes: (i) controlling the pumping system and the valve system to prevent fluid flow into and from the processing chamber, (ii) controlling the centrifuge at a selected rate and/or a selected relative centrifugal force; (iii) after a selected time, ending the flow rate stoppage phase; and (iv) resuming the interrupted stage or advancing to a subsequent stage of the blood separation procedure after ending the flow rate stoppage phase.

These and other aspects of the present subject matter are set forth in the following detailed description of the accompanying drawings.

BRIEF DESCRIPTION OF THE DRAWINGS

Fig. 1 is a perspective view of an exemplary reusable hardware component of a blood processing system which is configured to receive a disposable fluid flow circuit;

Fig. 2 is a plan view of an exemplary disposable fluid flow circuit for use in combination with the durable hardware component of Fig. 1;

Fig. 3 is a schematic view of the fluid flow circuit of Fig. 2 mounted to the processing device of Fig. 1 to complete a blood processing system according to an aspect of the present disclosure;

Fig. 4 is a schematic view of the blood processing system of Fig. 3 executing a "blood prime" stage of an exemplary blood processing procedure;

Fig. 5A is a schematic view of the blood processing system of Fig. 3 executing a "flow rate stoppage" phase of an exemplary blood processing procedure;

Fig. 5B is a flowchart illustrating one alternative of a logic or decisions to commence/continue a flow rate stoppage phase;

Fig. 5C is a flowchart illustrating another logic or decisions to commence/continue a flow rate stoppage phase;

Fig. 6 is a schematic view of the blood processing system of Fig. 3 executing an "establish separation" stage of an exemplary blood processing procedure;

Fig. 7 is a schematic view of the blood processing system of Fig. 3 executing a "collection" stage of an exemplary blood processing procedure, with separated red blood cells being leukoreduced before collection;

Fig. 8 is a schematic view of a variation of the "collection" stage of Fig. 7 in which the separated red blood cells are not leukoreduced before collection;

Fig. 9 is a schematic view of the blood processing system of Fig. 3 executing a "red blood cell recovery" stage of an exemplary blood processing procedure, with separated red blood cells being leukoreduced before collection;

Fig. 10 is a schematic view of a variation of the "red blood cell recovery" stage of Fig. 9 in which the separated red blood cells are not leukoreduced before collection;

Fig. 11 is a schematic view of the blood processing system of Fig. 3 executing an "additive solution flush" stage of an exemplary blood processing procedure, with additive solution being directed through a leukoreduction filter before entering a red blood cell collection container;

Fig. 12 is a schematic view of a variation of the "additive solution flush" stage of Fig. 11 in which the additive solution enters the red blood cell collection container without passing through the leukoreduction filter;

Fig. 13 is a schematic view of the blood processing system of Fig. 3 executing an "air evacuation" stage of an exemplary blood processing procedure; and

Fig. 14 is a schematic view of the blood processing system of Fig. 3 executing a "sealing" stage of an exemplary blood processing procedure.

DETAILED DESCRIPTION OF THE ILLUSTRATED EMBODIMENTS

The embodiments disclosed herein are for the purpose of providing an exemplary description of the present subject matter. They are, however, only exemplary and not exclusive, and the present subject matter may be embodied in various forms. Therefore, specific details disclosed herein are not to be interpreted as limiting the subject matter as defined in the accompanying claims.

The present disclosure is directed to devices and methods for whole blood separation using a centrifuge. The method includes one or more flow rate stoppage phases during any of the stages of the separation method. During a flow rate stoppage phase, flow of whole blood to the centrifuge and separated blood components from the centrifuge are stopped. While the flows of the whole blood and separated components are stopped, the centrifuge spins at a selected rate. For example, the centrifuge spins a rate between about 500 RPM and about 5500 RPM. In one alternative, the centrifuge spins at a rate of between about 1500 RPM and about 5000 RPM. In yet another alternative, the centrifuge spins at a rate of about 1500, or about 3500, or about 5000 RPM. Independent from or in addition to the spin rate, the relative centrifugal force (G) may be between about 10 Gs and about 1450 Gs. In other words, the relative centrifugal force may be within this range regardless of the spin rate or size of the centrifuge. In one alternative, the relative centrifugal force may be about 100Gs, and in another alternative about 1140Gs. After a selected time, the flow rate stoppage phase ends, flow of blood and blood components to and from the centrifuge resumes and the separation process continues. For example, the selected time may be between about 15 seconds and about 45 seconds. In one alternative, the selected time may be about 30 seconds.

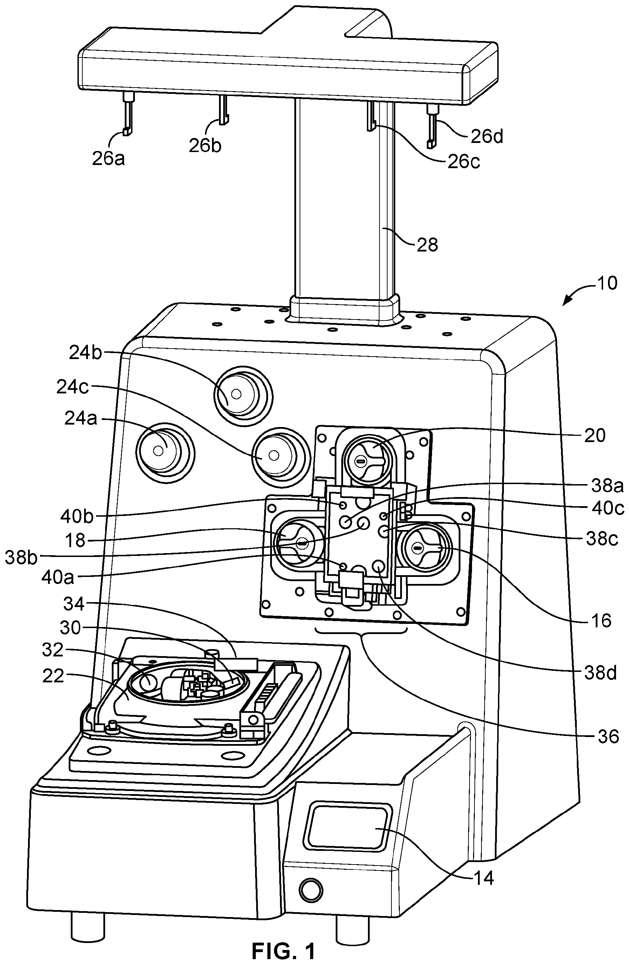

The method disclosed herein may be used with any suitable centrifugal blood separation system and/or process. The system and process described and shown relative to Figs. 1-14 are exemplary and are provided for the purpose of describing and understanding the method. Thus, it will be understood that this method is not limited to the blood separation systems and processes disclosed herein, and the method could be used with any suitable centrifugal blood separation system/process. Furthermore, the method disclosed herein can be used for separating whole blood and collection of its components, such as red blood cells, plasma, buffy coat, etc. For example, in one alternative, the method may be used to separate whole blood into red blood cells and plasma. In another alternative the system may be used to separate whole blood into red blood cells, plasma, and buffy coat.

The method includes flowing whole blood from a blood source to a centrifuge and spinning the centrifuge to separate the whole blood into its components. Optionally, the method also may include one or more of stages such as a blood priming stage, an establish separation stage, a collection stage, an air evacuation stage, an additive solution flush stage, or any other suitable stage or process. Periodically and at least once during any point in the method, a flow rate stoppage phase is initiated. During the flow rate stoppage phase, flow to and from the centrifuge is stopped and the blood within the centrifuge is spun at a desired rate for a desired period of time to allow cells to further separate from the plasma fraction of the blood. The flow stoppage phase may include stopping one or more pumps of a pumping system and/or closing one or more valves of a valve system.

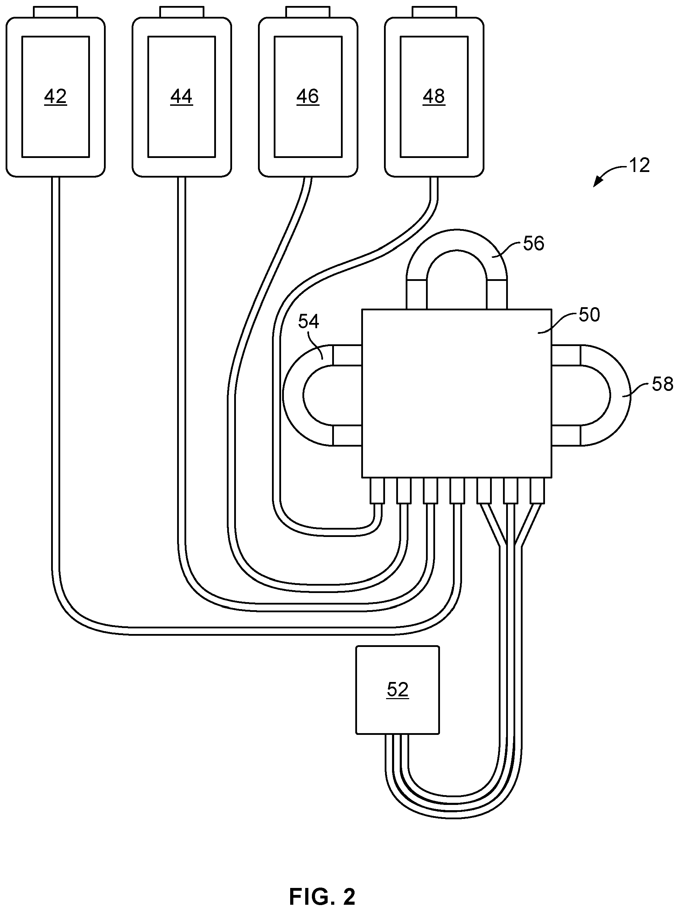

Turing now to Fig. 1, it depicts a reusable or durable hardware component or processing device of a configurable, automated blood processing system or blood component manufacturing system, generally designated 10, while Fig. 2 depicts a disposable or single use fluid flow circuit, generally designated 12, to be used in combination with the processing device 10 for processing collected whole blood. The illustrated processing device 10 includes associated pumps, valves, sensors, displays, and other apparatus for configuring and controlling flow of fluid through the fluid flow circuit 12, described in greater detail below. The blood processing system may be directed by a controller integral with the processing device 10 that includes a programmable microprocessor to automatically control the operation of the pumps, valves, sensors, etc. The processing device 10 may also include wireless communication capabilities to enable the transfer of data from the processing device 10 to the quality management systems of the operator.

More specifically, the illustrated processing device 10 includes a user input and output touchscreen 14, a pump station or system including a first pump 16 (for pumping, e.g., whole blood), a second pump 18 (for pumping, e.g., plasma) and a third pump 20 (for pumping, e.g., additive solution), a centrifuge mounting station and drive unit 22 (which may be referred to herein as a "centrifuge"), and a valve system which includes any suitable type of valves, such as clamps 24a-c. The touchscreen 14 enables user interaction with the processing device 10, as well as the monitoring of procedure parameters, such as flow rates, container weights, pressures, etc. The pumps 16, 18, and 20 (collectively referred to herein as being part of a "pump system" of the processing device 10) are illustrated as peristaltic pumps capable of receiving tubing or conduits and moving fluid at various rates through the associated conduit dependent upon the procedure being performed. An exemplary centrifuge mounting station/drive unit is seen in U.S. Patent No. 8,075,468 (with reference to Figs. 26-28), which is hereby incorporated herein by reference. The clamps 24a-c (collectively referred to herein as being part of the "valve system" of the processing device 10) are capable of opening and closing fluid paths through the tubing or conduits and may incorporate RF sealers in order to complete a heat seal of the tubing or conduit placed in the clamp to seal the tubing or conduit leading to a product container upon completion of a procedure.

Sterile connection/docking devices may also be incorporated into one or more of the clamps 24a-c. The sterile connection devices may employ any of several different operating principles. For example, known sterile connection devices and systems include radiant energy systems that melt facing membranes of fluid flow conduits, as in U.S. Patent No. 4,157,723; heated wafer systems that employ wafers for cutting and heat bonding or splicing tubing segments together while the ends remain molten or semi-molten, such as in U.S. Patent Nos. 4,753,697; 5,158,630; and 5,156,701; and systems employing removable closure films or webs sealed to the ends of tubing segments as described, for example, in U.S. Patent No. 10,307,582. Alternatively, sterile connections may be formed by compressing or pinching a sealed tubing segment, heating and severing the sealed end, and joining the tubing to a similarly treated tubing segment as in, for example, U.S. Patent Nos. 10,040,247 and 9,440,396. All of the above-identified patents are incorporated by reference in their entirety. Sterile connection devices based on other operating principles may also be employed without departing from the scope of the present disclosure.

The processing device 10 also includes hangers 26a-d (which may each be associated with a weight scale) for suspending the various containers of the disposable fluid circuit 12. The hangers 26a-d are preferably mounted to a support 28, which is vertically translatable to improve the transportability of the processing device 10. An optical system comprising a laser 30 and a photodetector 32 is associated with the centrifuge 22 for determining and controlling the location of an interface between separated blood components within the centrifuge 22. An exemplary optical system is shown in U.S. Patent Application Publication No. 2019/0201916, which is hereby incorporated herein by reference. An optical sensor 34 is also provided to optically monitor one or more conduits leading into or out of the centrifuge 22.

The face of the processing device 10 includes a nesting module 36 for seating a flow control cassette 50 (Fig. 2) of the fluid flow circuit 12 (described in greater detail below). The cassette nesting module 36 is configured to receive various disposable cassette designs so that the system may be used to perform different types of procedures. Embedded within the illustrated cassette nesting module 36 are four valves 38a-d (collectively referred to herein as being part of the "valve system" of the processing device 10) for opening and closing fluid flow paths within the flow control cassette 50, and three pressure sensors 40a-c capable of measuring the pressure at various locations of the fluid flow circuit 12.

With reference to Fig. 2, the illustrated fluid flow circuit 12 includes a plurality of containers 42, 44, 46, and 48, with a flow control cassette 50 and a processing/separation chamber 52 that is configured to be received in the centrifuge 22 (the processing chamber 52 and centrifuge 22 may be collectively referred to as the "centrifuge assembly"), all of which are interconnected by conduits or tubing segments, so as to permit continuous flow centrifugation. The flow control cassette 50 routes the fluid flow through three tubing loops 54, 56, 58, with each loop being positioned to engage a particular one of the pumps 16, 18, 20. The conduits or tubing may extend through the cassette 50, or the cassette 50 may have pre-formed fluid flow paths that direct the fluid flow.

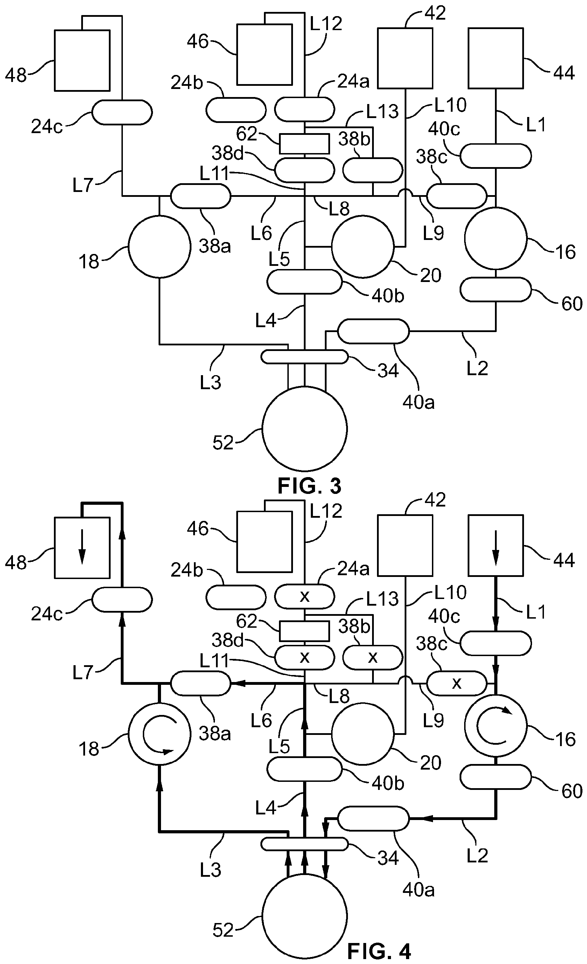

In the fluid flow circuit 12 shown in Fig. 2, container 42 may be pre-filled with additive solution, container 44 may be filled with whole blood and connected to the fluid flow circuit 12 at the time of use, container 46 may be an empty container for the receipt of red blood cells separated from the whole blood, and container 48 may be an empty container for the receipt of plasma separated from the whole blood. While Fig. 2 shows a whole blood container 44 (configured as a blood pack unit, for example) as a blood source, it is within the scope of the present disclosure for the blood source to be a living donor, as will be described in greater detail herein. The fluid flow circuit may optionally include an air trap 60 (Fig. 3) through which the whole blood is flowed prior to entering the separation chamber and/or a leukoreduction filter 62 through which the red blood cells are flowed prior to entering the red blood cell collection container 46.

The processing chamber 52 may be pre-formed in a desired shape and configuration by injection molding from a rigid plastic material, as shown and described in U.S. Patent No. 6,849,039, which is hereby incorporated herein by reference. The specific geometry of the processing chamber 52 may vary depending on the elements to be separated, and the present disclosure is not limited to the use of any specific chamber design. For example, it is within the scope of the present disclosure for the processing chamber 52 to be configured formed of a generally flexible material, rather than a generally rigid material. When the processing chamber 52 is formed of a generally flexible material, it relies upon the centrifuge 22 to define a shape of the processing chamber 52. An exemplary processing chamber formed of a flexible material and an associated centrifuge are described in U.S. Patent No. 6,899,666, which is hereby incorporated herein by reference.

In keeping with the disclosure, the controller of the processing device 10 is pre-programmed to automatically operate the system to perform one or more standard blood processing procedures selected by an operator by input to the touchscreen 14, and configured to be further programmed by the operator to perform additional blood processing procedures. The controller may be pre-programmed to substantially automate a wide variety of procedures, including, but not limited to: red blood cell and plasma production from a single unit of whole blood (as will be described in greater detail herein), buffy coat pooling, buffy coat separation into a platelet product (as described in U.S. Patent Application Publication No. 2018/0078582, which is hereby incorporated herein by reference), glycerol addition to red blood cells, red blood cell washing, platelet washing, and cryoprecipitate pooling and separation.

The pre-programmed blood processing procedures operate the system at pre-set settings for flow rates and centrifugation forces, and the programmable controller may be further configured to receive input from the operator as to one or more of flow rates and centrifugation forces for the standard blood processing procedure to override the pre-programmed settings.

In addition, the programmable controller is configured to receive input from the operator through the touchscreen 14 for operating the system to perform a non-standard blood processing procedure. More particularly, the programmable controller may be configured to receive input for settings for the non-standard blood processing procedure, including flow rates and centrifugation forces.

Red Blood Cell And Plasma Product Collection

In an exemplary procedure, the processing device 10 and the fluid flow circuit 12 may be used in combination to process a unit of whole blood into a red blood cell product and a plasma product in accordance with the method disclosed herein. Fig. 3 is a schematic illustration of the exemplary fluid flow circuit 12 mounted to the processing device 10, with selected components of the fluid flow circuit 12 and selected components of the processing device 10 being shown. Figs. 4-14 show different stages of an exemplary procedure. As shown in Figs. 3-14, one of the clamps 24b is not used in producing the red blood cell and plasma products (but could be used in other procedures), while the other illustrated components of the processing device 10 are employed.

In an initial stage, which is referred to herein as a "blood prime" stage and shown in Fig. 4, selected components of the fluid flow circuit 12 are primed using blood from a blood source. This is in contrast to typical apheresis devices, which employ a separately provided fluid (e.g., anticoagulant or saline) to prime a fluid flow circuit. The blood source is shown in Fig. 4 as the whole blood container 44, but may alternatively be a living donor. Thus, it should be understood that the term "whole blood" may refer to blood that either includes or omits an anticoagulant fluid.

During the blood prime stage, whole blood is drawn into the fluid flow circuit 12 from the blood source (the whole blood container 44 in the embodiment of Fig. 4) via line L1 by operation of the first pump 16 (which may be referred to as the "whole blood pump"). Valve 38c is closed, which directs the blood through pressure sensor 40c and into line L2. The blood passes through air trap 60, pressure sensor 40a (which measures the pressure of the processing chamber 52), and optical sensor 34 before flowing into the processing chamber 52, which is positioned within the centrifuge 22 of the processing device 10.

The centrifuge 22 may be stationary during the blood prime stage or may instead be controlled by the controller of the processing device 10 to spin at a low rotation rate (e.g., on the order of approximately 1,000-2,000 rpm). It may be advantageous for the centrifuge 22 to rotate during the blood prime stage in order to create enough g-force to ensure that the air in the processing chamber 52 (which includes air already present in the processing chamber 52, along with air moved into the processing chamber 52 from lines L1 and/or L2 by the flow of blood) is forced towards the low-g (radially inner) wall of the processing chamber 52. Higher centrifuge rotation rates, such as 4,500 rpm (which is required for steady state separation, as will be described) may be undesirable as air blocks (in which air gets stuck and cannot be forced out of the processing chamber 52, causing pressure to rise) are more likely at higher g-forces.

The blood entering the processing chamber 52 will move towards the high-g (radially outer) wall of the processing chamber 52, displacing air towards the low-g wall. A plasma outlet port of the processing chamber 52 is associated with the low-g wall of the processing chamber 52, such that most of the air will exit the processing chamber 52 via the plasma outlet port and associated line L3, although some air may also exit the processing chamber 52 via a red blood cell outlet port associated with the high-g wall of the processing chamber 52.

Valves 38b and 38d are closed, while the second pump 18 (which may be referred to as the "plasma pump") is active and the third pump 20 (which may be referred to as the "additive pump") is inactive. Such an arrangement will direct the air exiting the processing chamber 52 via the red blood cell outlet port through associated line L4 and pressure sensor 40b, into line L5 and then into line L6. Valve 38a is open, such that the air flowing through line L6 will meet up with the air flowing through line L3 (i.e., the air that exits the processing chamber 52 via the plasma outlet port). The combined air will flow through line L7 and open clamp 24c, into the plasma collection container 48. It should be understood that, in Figs. 4-14, arrows on the containers represent the direction of fluid flow between the container and the conduit connected to the container and the absence of arrows represents no fluid flow through the respective conduit. For example, line L7 is shown as being connected to the top of the plasma collection container 48, such that a downward arrow (as in Fig. 4) represents downward fluid flow into the plasma collection container 48. In contrast, line L1 is shown as being connected to the bottom of the whole blood container 44, such that a downward arrow (as in Fig. 4) represents downward fluid flow out of the whole blood container 44.

The flow of air out of the processing chamber 52 via either outlet port is monitored by the optical sensor 34, which is capable of determining the optical density of the fluid flowing through the monitored lines and discerning between air and a non-air fluid in lines L3 and L4. When a non-air fluid is detected in both lines L3 and L4, the controller of the processing device 10 will end the blood prime stage and move on to the next stage of the procedure. The amount of blood drawn into the fluid flow circuit 12 from the blood source during the blood prime stage will vary depending on a number of factors (e.g., the amount of air in the fluid flow circuit 12), but may be on the order of approximately 50 to 100 mL. The blood prime stage may take on the order of one to two minutes.

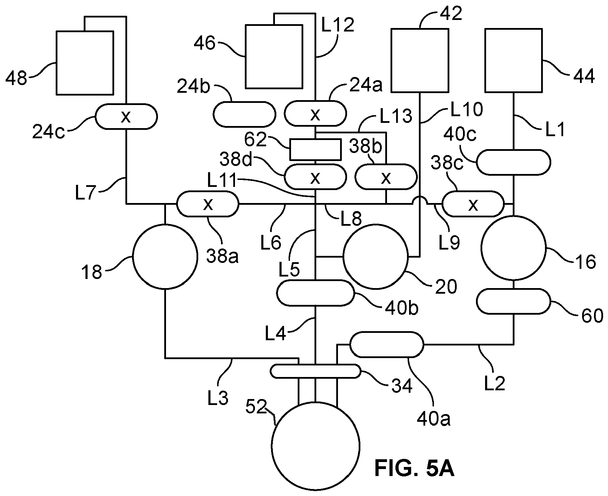

Referring to Fig. 5A, as mentioned above, a "flow rate stoppage phase" may be executed one or more times during the blood separation and collection process. Additionally, the flow rate stoppage phase may be executed periodically during or after any of the stages of the separation and collection process. Optionally, during or after the priming stage, the flow rate stoppage phase is executed. During such phase, flow of whole blood to the centrifuge assembly (processing chamber 52 and centrifuge 22) is stopped and flow of fluid from the centrifuge assembly is stopped. The flow stoppage may be achieved by stopping or deactivating one or more of the pumps 16, 18 and 20 of the pumping system, and/or closing one or more of the valves 24a, 24c and 38a-38d of the valve system. For example, in Fig. 5A, pumps 16, 18 and 20 are stopped and valves 24a, 24c and 38a-38d are closed. This prevents whole blood from flowing into the centrifuge assembly and prevents the flow of fluid, such as blood components, out of the centrifuge assembly.

During the flow rate stoppage phase, the centrifuge 22 is spun at a selected rate and/or at a selected centrifugal force (G). For example, the centrifuge spins at a rate between about 500 RPM and about 5500 RPM. In one alternative, the centrifuge spins at a rate of between about 1500 RPM and about 5000 RPM. In yet another alternative, the centrifuge spins at a rate of about 1500, or about 3500, or about 5000 RPM. Independent from or in addition to the spin rate, the relative centrifugal force may be between about 10 Gs and about 1450 Gs. In one alternative, the relative centrifugal force may be 100Gs, and in another alternative about 1140Gs. After a selected time, flow of blood and blood components to and from the centrifuge assembly resumes and the blood separation method continues. For example, at the end of the selected time, the flow rate stoppage phase ends and one or more of pumps 16, 18, 20 may be activated and one or more of the valves 24a, 24c and 38a-38d may be opened to resume flow. When flow resumes, the current stage may resume or the method may be moved on to the next stage of the method. In one alternative, the selected time may be between about 15 seconds and about 45 seconds. In one alternative, the selected time may be about 30 seconds.

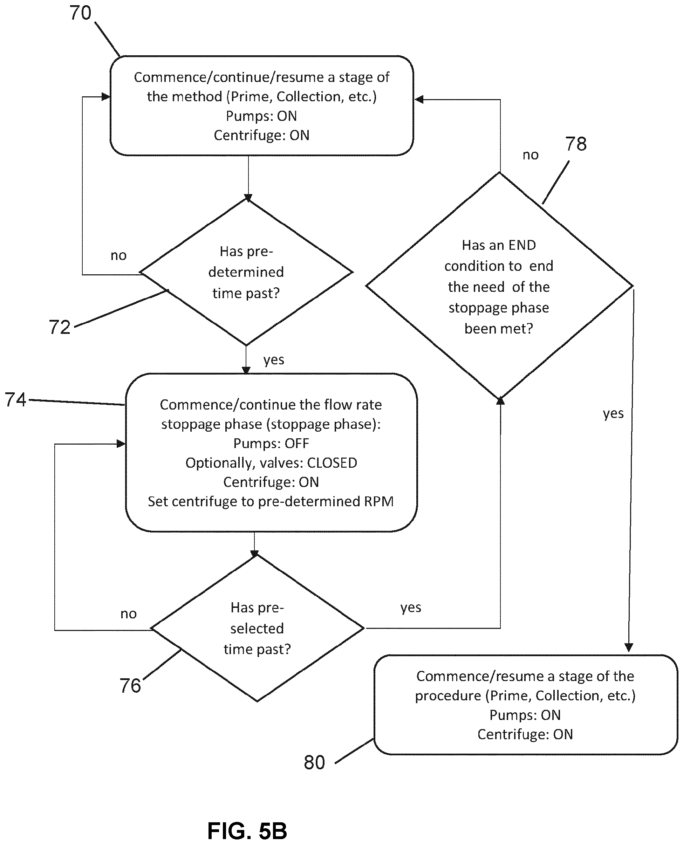

Fig. 5B includes a flow chart illustrating one alternative decision tree for executing a flow rate stoppage phase or periodic flow rate stoppage phases. At 70, a stage, such as any of the stages disclosed herein (prime, establish separation, collection, etc.) has commenced or is continued/resumed. At this time, pumps may be activated/on and the centrifuge is on. Turning to 72, if a predetermined time from the commencement/continuation/resumption of the stage has passed, it is possible for the flow rate stoppage phase 74 to be commenced/executed; otherwise, if the predetermined time has not passed at 72, then the stage continues at 70. During the flow rate stoppage phase, pumps may be off and, optionally, valves may be closed, and the centrifuge is set to spin at a selected RPM and/or relative centrifugal force. After a flow rate stoppage phase has started, it is determined at 76 whether a selected time has passed (such as any of the selected times discussed above). If no, then the flow rate stoppage phase continues at 74; otherwise, if the selected time has passed, then it is determined at 78 if an END condition has occurred. Such END conditions may include, but are not limited to, a selected number of flow stoppage phases have been completed, a specific volume of blood has been processed, or a stage has ended. If the END condition has not occurred, then the stage resumes at 70 and the process repeats as described above. Otherwise, if the END conditions has occurred, then at 80 the stage resumes with no further flow rate stoppage phases or the next stage begins.

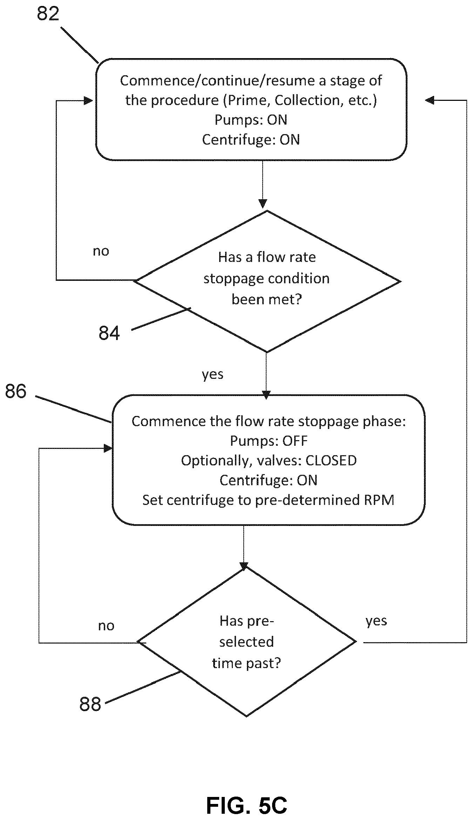

Fig. 5C includes a flow chart illustrating an alternative decision tree for executing flow rate stoppage phases. At 82, a stage has commenced or is continued/resumed. At this time, pumps may be activated/on and the centrifuge is on. Turning to 84, it is determined if a flow rate stoppage condition has been met. Such flow rate stoppage conditions may include, but are not limited to, the start or end of a stage (prime, establish, collection), a specific volume of blood has been processed during the stage, or an optical measurement of a centrifuge exit line indicating unwanted fluid content (e.g., platelets in the PPP line). If the flow rate stoppage condition has been met, it is possible for the flow rate stoppage phase 86 to be commenced/executed; otherwise, if the condition has not been met, then the stage continues at 82. During the flow rate stoppage phase, pumps may be off and, optionally, valves may be closed, and the centrifuge is set to spin at a selected RPM and/or relative centrifugal force. After a flow rate stoppage phase has started, it is determined at 88 whether a selected time has passed. If no, then the flow rate stoppage phase continues at 86; otherwise, if the selected time has passed, then the stage resumes or a new stage commences at 82 and the process repeats as described above.

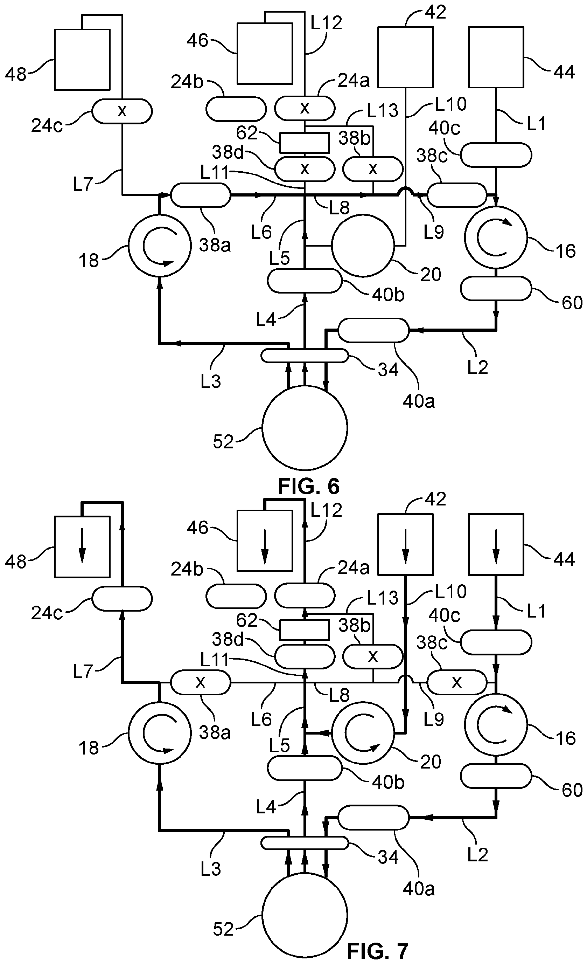

The next stage (shown in Fig. 6) is referred to herein as the "establish separation" stage. Once non-air fluid has been detected in lines L3 and L4, the rotational speed of the centrifuge 22 will be increased to a rate that is sufficient to separate blood into packed red blood cells and platelet-poor plasma (which may be in the range of approximately 4,500 to 5,500 rpm, for example). To produce a plasma product that is low in platelets, it may be advantageous for the processing chamber 52 to be configured with a plasma outlet port that is spaced from and positioned downstream of the blood inlet port, rather than being positioned adjacent to the blood inlet port. Such a configuration allows the platelets to settle down into a distinct layer between the plasma and the red blood cells (commonly referred to as a "buffy coat") before the plasma is removed from the processing chamber 52, thus allowing the separated plasma to be platelet-depleted. As for the whole blood pump 16, it continues to operate, but no additional blood is drawn into the fluid flow circuit 12 from the blood source during the establish separation stage (as will be described).

As the blood source includes (in the case of a whole blood container) or provides (in the case of a living donor) only a single unit of whole blood (approximately 500 mL), the system must work with a finite fluid volume. To avoid product loss or quality issues, the plasma and red blood cells initially separated from the blood in the processing chamber 52 and removed from the processing chamber 52 are not directed to their respective collection containers, but are instead mixed together to form recombined whole blood and recirculated back into the processing chamber 52.

More particularly, during the establish separation stage, separated plasma will exit the processing chamber 52 via the plasma outlet port and associated line L3. Clamp 24c is closed during this stage, while valve 38a remains open, which directs the plasma from line L3 into line L6. Separated red blood cells exit the processing chamber 52 via the red blood cell outlet port and associated line L4. In the illustrated embodiment, there is no pump associated with line L4, such that the red blood cells exit the processing chamber 52 at a rate that is equal to the difference between the rate of the whole blood pump 16 and the rate of the plasma pump 18. In alternative embodiments, there may be a pump associated with the red blood cell outlet line instead of the plasma outlet line or a first pump associated with the plasma outlet line and a second pump associated with the red blood cell outlet line.

The additive pump 20 is inactive during this stage, thereby directing the red blood cells from line L4 into line L5. The plasma flowing through line L6 is mixed with the red blood cells flowing through line L5 at a junction of the two lines L5 and L6 to form recombined whole blood. Valve 38d is closed, which directs the recombined whole blood into line L8. Valve 38b is also closed, which directs the recombined whole blood from line L8 into line L9 and through open valve 38c. The whole blood pump 16 draws the recombined whole blood into line L2 from line L9 (rather than drawing additional blood into the fluid flow circuit 12 from the blood source), with the recombined blood passing through air trap 60, pressure sensor 40a, and optical sensor 34 before flowing back into the processing chamber 52, where it is again separated into plasma and red blood cells.

The establish separation stage continues until steady state separation has been achieved, which may take on the order of approximately one to two minutes. As used herein, the phrase "steady state separation" refers to a state in which blood is separated into its constituents in the processing chamber 52, with the radial position of the interface between separated components within the processing chamber 52 being at least substantially maintained (rather than moving radially inwardly or outwardly). The position of the interface may be determined and controlled according to any suitable approach, including using an interface detector of the type described in U.S. Patent Application Publication No. 2019/0201916.

Preferably, steady state separation is achieved with the interface between separated components within the processing chamber 52 at a target location. The target location may correspond to the location of the interface at which separation efficiency is optimized, with the precise location varying depending on a number of factors (e.g., the hematocrit of the whole blood). However, in an exemplary embodiment, the target location of the interface may be the position of the interface when approximately 52% of the thickness or width (in a radial direction) of the channel defined by the processing chamber 52 is occupied by red blood cells. In the illustrated embodiment, the position of the interface within the processing chamber 52 may be adjusted by changing the flow rate of the plasma pump 18, with the flow rate being increased to draw more separated plasma out of the processing chamber 52 (which decreases the thickness of the plasma layer within the processing chamber 52) and move the interface toward the low-g wall or decreased to draw less plasma out of the processing chamber 52 (which increases the thickness of the plasma layer within the processing chamber 52) and move the interface toward the high-g wall.

In an exemplary procedure, the controller of the processing device 10 will control the whole blood pump 16 to operate at a constant rate, with the plasma pump 18 initially operating at the same rate, which will quickly increase the thickness of the red blood cell layer within the processing chamber 52 and move the interface toward the low-g wall. The rate of the plasma pump 18 is gradually decreased as the thickness of the red blood cell layer increases and the location of the interface approaches the target location. As described above, the target location of the interface may depend upon the hematocrit of the whole blood, meaning that the rate of the plasma pump 18 (which controls the position of the interface) may also depend on the hematocrit of the whole blood. In one embodiment, this relationship may be expressed as follows:

$$\text{Theoretical plasma pump rate} = \text{whole blood pump rate} - ((\text{whole blood hematocrit} * \text{whole blood pump rate}) / \text{hematocrit of separated red blood cells}) \ [\text{Equation 1}]$$

The hematocrit of the whole blood may be measured before the procedure begins or by the optical sensor 34 during the procedure, while the hematocrit of the separated red blood cells may be determined during the procedure by the optical sensor 34 monitoring line L4. In practice, the plasma pump rate will typically not remain at the theoretical rate once steady state separation has been achieved, with the interface at the target location, but rather the plasma pump rate will instead tend to "flutter" around the theoretical rate.

Referring back to Fig. 5A, optionally, a flow rate stoppage phase may be executed one or more times during the establish separation stage. During such phase, flow of whole blood to the centrifuge assembly (processing chamber 52 and centrifuge 22) is stopped and flow of fluid from the centrifuge assembly is stopped. The flow stoppage may occur by stopping or deactivating one or more of the pumps 16, 18 and 20 of the pumping system, and/or closing one or more of the valves 24a, 24c and 38a-38d of the valve system.

During flow rate stoppage phase, the centrifuge 22 is spun at a selected rate and/or a selected relative centrifugal force. For example, the centrifuge spins a rate between about 500 RPM and about 5500 RPM. In one alternative, the centrifuge spins at a rate of between about 500 RPM and about 5500 RPM. In another alternative, the centrifuge spins at a rate of between about 1500 RPM and about 5000 RPM. In yet another alternative, the centrifuge spins at a rate of about 1500, or about 3500, or about 5000 RPM. Independent from or in addition to the spin rate, the relative centrifugal force may be between about 10 Gs and about 1450 Gs. In one alternative, the relative centrifugal force may be 100Gs, and in another alternative about 1140Gs. After a selected time, flow of blood and blood components to and from the centrifuge assembly resumes and the method continues. For example, at the end of the selected time, the flow rate stoppage phase ends and one or more of pumps 16, 18, 20 may be activated and one or more of the valves 24a, 24c and 38a-38d may be opened to resume flow. When flow resumes, the establish separation stage may resume or the procedure may be moved on to the next stage. In one alternative, the selected time may be between about 15 seconds and about 45 seconds. In one alternative, the selected time may be about 30 seconds.

Regardless of the particular manner in which the controller of the processing device 10 executes the establish separation stage and arrives at steady state separation, once steady state separation has been established, the controller ends the establish separation stage and advances the procedure to a "collection" stage, which is illustrated in Fig. 7. At the beginning of the collection stage, the centrifuge 22, the whole blood pump 16, and the plasma pump 18 all continue operating at the same rates at which they were operating at the end of the establish separation stage. The valve system of the processing device 10, however, is adjusted to direct the separated plasma and red blood cells to their respective collection containers (rather than recombining them and recirculating them through the centrifuge 22), while causing additional blood to be drawn into the fluid flow circuit 12 from the blood source until a total of one unit of whole blood has been drawn into the fluid flow circuit 12.

More particularly, during the collection stage, valve 38c is closed, which causes the whole blood pump 16 to draw additional blood into line L1 from the blood source (which is the whole blood container 44 in the illustrated embodiment, but may be a living donor). The whole blood pump 16 draws the blood from the blood source into line L2 from line L1, with the blood passing through air trap 60, pressure sensor 40a, and optical sensor 34 before flowing into the processing chamber 52, where it is separated into plasma and red blood cells. Most of the platelets of the whole blood will remain in the processing chamber 52, along with some white blood cell populations (much as mononuclear cells), while larger white blood cells, such as granulocytes, may exit with the packed red blood cells.

The separated plasma exits the processing chamber 52 via the plasma outlet port and associated line L3. Valve 38a is closed, which directs the plasma from line L3 into line L7, through open clamp 24c, and into the plasma collection container 48.

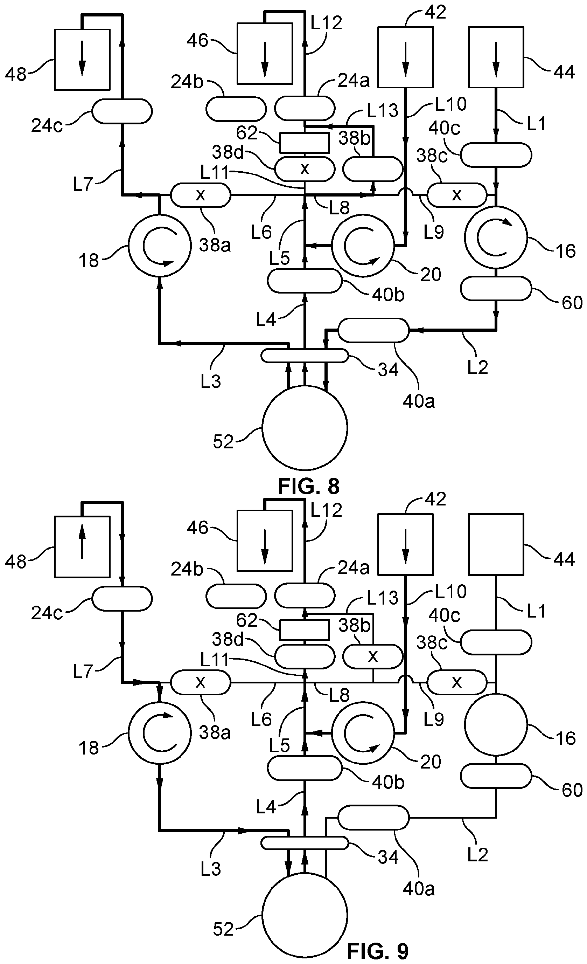

As for the separated red blood cells, they exit the processing chamber 52 via the red blood cell outlet port and associated line L4. The additive pump 20 is operated by the controller to draw an additive solution (which is ADSOLO in one exemplary embodiment, but may be some other red blood cell additive) from the additive solution container 42 via line L10. The red blood cells flowing through line L4 are mixed with the additive solution flowing through line L10 at a junction of the two lines L4 and L10 to form a mixture that continues flowing into and through line L5. The mixture is ultimately directed into the red blood cell collection container 46, but may first be conveyed through a leukoreduction filter 62 (if provided), as shown in Fig. 7. Even if a leukoreduction filter 62 is provided, the valve system may be controlled to cause the mixture to bypass the leukoreduction filter 62 and enter the red blood cell collection container 46 without being leukoreduced, as shown in Fig. 8. It is also within the scope of the present disclosure for the mixture to be routed through the leukoreduction filter 62 at the beginning of the collection stage, with the valve system being reconfigured during the collection stage to cause the mixture to bypass the leukoreduction filter 62, such that only a portion of the collected red blood cells are leukoreduced.

In the configuration of Fig. 7 (in which the mixture is leukoreduced), valves 38a, 38b, and 38c are closed, while valve 38d is open, which directs the mixture from line L5 into line L11. The mixture flows through open valve 38d and the leukoreduction filter 62 and into line L12. The leukoreduced mixture then flows through open clamp 24a and into the red blood cell collection container 46.

In the configuration of Fig. 8 (in which the mixture is not leukoreduced), valves 38a, 38c, and 38d are closed, while valve 38b is open, which directs the mixture from line L5 into line L8 and then into line L13. The mixture flows through open valve 38b and into line L12, bypassing the leukoreduction filter 62. The non-leukoreduced mixture then flows through open clamp 24a and into the red blood cell collection container 46.

As described above, the mixture may be routed through the leukoreduction filter 62 at the beginning of the collection stage (as in Fig. 7), with the valve system being reconfigured during the collection stage to cause the mixture to bypass the leukoreduction filter 62 (as in Fig. 8), such that only a portion of the collected red blood cells are leukoreduced. In one embodiment, pressure sensor 40b monitors the pressure of the leukoreduction filter 62. If the pressure sensor 40b detects that the pressure of the leukoreduction filter 62 has risen above a predetermined pressure threshold (which may be indicative of filter blockage), the controller may reconfigure the valve system (from the configuration of Fig. 7 to the configuration of Fig. 8) to cause the mixture to bypass the leukoreduction filter 62. The system may then alert the operator that the red blood cell product was not leukoreduced.

Regardless of whether the collected red blood cells have been leukoreduced (or only partially leukoreduced), the collection stage continues until one unit of whole blood has been drawn into the fluid flow circuit 12 from the blood source. In the case of a whole blood container 44 being used as a blood source (as in the illustrated embodiment) the collection stage will end when the whole blood container 44 (which is initially provided with one unit of whole blood) is empty, with different approaches possibly being employed to determine when the whole blood container 44 is empty. For example, in one embodiment, pressure sensor 40c monitors the hydrostatic pressure of the whole blood container 44. An empty whole blood container 44 may be detected when the hydrostatic pressure measured by pressure sensor 40c is at or below a threshold value. Alternatively

(or additionally), the weight of the whole blood container 44 may be monitored by a weight scale, with an empty whole blood container 44 being detected when the weight is at or below a threshold value. In the case of a living donor (or in the event that the whole blood container 44 is provided with more than one unit of blood), the volumetric flow rate of the whole blood pump 16 may be used to determine when one unit of whole blood has been drawn into the fluid flow circuit 12.

Referring back to Fig. 5A, optionally, a flow rate stoppage phase may be executed one or more times during the collection stage. During such phase flow of whole blood to the centrifuge assembly (processing chamber 52 and centrifuge 22) is stopped and flow of fluid from the centrifuge assembly is stopped. The flow stoppage may occur by stopping or deactivating one or more of the pumps 16, 18 and 20 of the pumping system, and/or closing one or more of the valves 24a, 24c and 38a-38d of the valve system.

During flow rate stoppage phase, the centrifuge 22 is spun at a selected rate and/or a selected relative centrifugal force. For example, the centrifuge spins a rate between about 500 RPM and about 5500 RPM. In one alternative, the centrifuge spins at a rate of between about 1500 RPM and about 5000 RPM. In yet another alternative, the centrifuge spins at a rate of about 1500, or about 3500, or about 5000 RPM. Independent from or in addition to the spin rate, the relative centrifugal force (G) may be between about 10 Gs and about 1450 Gs. In one alternative, the relative centrifugal force may be 100Gs, and in another alternative about 1140Gs. After a selected time, flow of blood and blood components to and from the centrifuge assembly resumes and the method continues. For example, at the end of the selected time, the flow rate stoppage phase ends and one or more of pumps 16, 18, 20 may be activated and one or more of the valves 24a, 24c and 38a-38d may be opened to resume flow. When flow resumes, the collection stage may resume or the procedure may be moved on to the next stage. In one alternative, the selected time may be between about 15 seconds and about 45 seconds. In one alternative, the selected time may be about 30 seconds.

Once a total of one unit of whole blood has been drawn into the fluid flow circuit 12, the controller will transition the procedure to a "red blood cell recovery" stage, which is shown in Fig. 9. During the red blood cell recovery stage, air from the plasma collection container 48 (which was conveyed there during the blood prime stage) is used to recover the contents of the processing chamber 52 (which may be primarily red blood cells) to reduce product loss.

In the illustrated embodiment, the whole blood pump 16 is deactivated, while the plasma pump 18 is operated in a reverse direction (with respect to its direction of operation up to this stage of the procedure). This draws the air from the plasma collection container 48 and into line L7. Valve 38a is closed, while clamp 24c is open, which directs the air through line L7, into and through line L3, and into the processing chamber 52 via the plasma outlet port. On account of the air flowing through the plasma outlet port, it will enter the processing chamber 52 at the low-g side. As additional air is introduced into the processing chamber 52, it will move from the low-g wall towards the high-g wall, thus displacing any liquid content through the red blood cell outlet port at the high-g side and into line L4. During this stage, the centrifuge 22 may be operated at a slower rate (e.g., in the range of approximately 1,000-2,000 rpm) to decrease the risk of an air blockage (as during the blood prime stage).

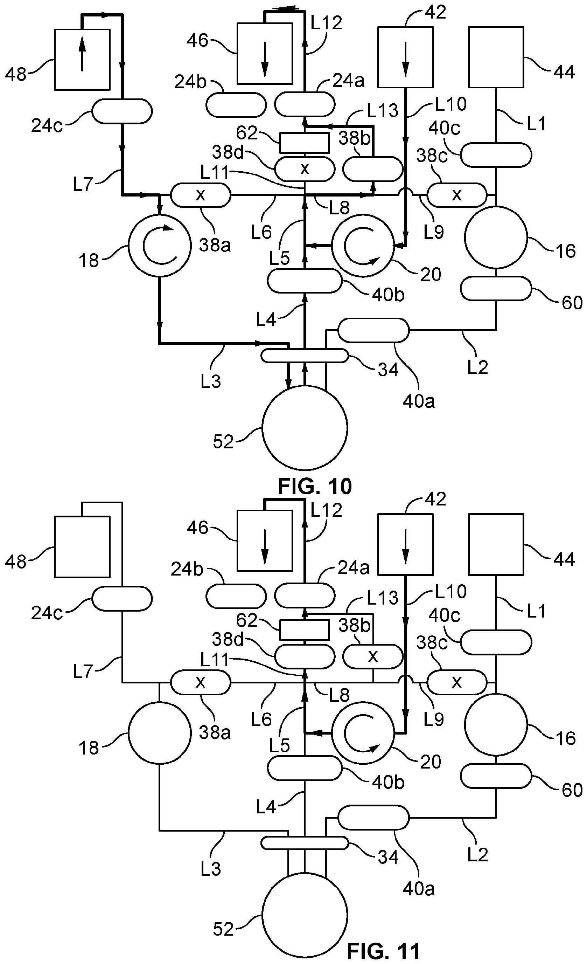

The additive pump 20 continues its operation, drawing additive solution from the additive solution container 42 and through line L10, to be mixed with the contents of the processing chamber 52 flowing through line L4 at the junction of the two lines L4 and L10. The mixture continues flowing into and through line L5. If the valve system was arranged in the configuration of Fig. 7 at the end of the collection stage (so as to direct flow through the leukoreduction filter 62), valves 38a, 38b, and 38c may remain closed, with valve 38d being open to direct the mixture into line L11 for leukoreduction, as in Fig. 9. On the other hand, if the valve system was arranged in the configuration of Fig. 8 at the end of the collection stage (so as to bypass the leukoreduction filter 62), valves 38a, 38c, and 38d may remain closed, with valve 38b being open to direct the mixture through lines L8 and L13 to bypass the leukoreduction filter 62, as in Fig. 10. As described above with regard to the collection stage, it is possible for the controller to change the configurations of the valve system from the configuration shown in Fig. 9 to the configuration of Fig. 10 during the red blood cell recovery stage to stop leukoreduction of the mixture (e.g., if the pressure of the leukoreduction filter 62 becomes too great).

Regardless of whether the mixture is filtered, it flows into line L12, through open clamp 24a, and into the red blood cell collection container 46. The red blood cell recovery stage continues until all of the air is removed from the plasma collection container 48. In one exemplary embodiment, the weight of the plasma collection container 48 may be monitored by a weight scale, with an empty plasma collection container 48 being detected when the weight is at or below a threshold value. Other approaches may also be employed to determine when to end the red blood cell recovery stage, such as using the optical sensor 34 to detect plasma flowing through line L3.

Referring back to Fig. 5A, optionally, a flow rate stoppage phase may be executed one or more times during the red blood cell recovery stage. During such phase, flow of whole blood to the centrifuge assembly (processing chamber 52 and centrifuge 22) is stopped and flow of fluid from the centrifuge assembly is stopped. The flow stoppage may occur by stopping or deactivating one or more of the pumps 16, 18 and 20 of the pumping system, and/or closing one or more of the valves 24a, 24c and 38a-38d of the valve system.

During flow rate stoppage phase, the centrifuge 22 is spun at a selected rate and/or a selected relative centrifugal force. For example, the centrifuge spins a rate between about 500 RPM and about 5500 RPM. In one alternative, the centrifuge spins at a rate of between about 1500 RPM and about 5000 RPM. In yet another alternative, the centrifuge spins at a rate of about 1500, or about 3500, or about 5000 RPM. Independent from or in addition to the spin rate, the relative centrifugal force may be between about 10 Gs and about 1450 Gs. In one alternative, the relative centrifugal force may be 100Gs, and in another alternative about 1140Gs. After a selected time, flow of blood and blood components to and from the centrifuge assembly resumes and the method continues. For example, at the end of the selected time, the flow rate stoppage phase ends and one or more of pumps 16, 18, 20 may be activated and one or more of the valves 24a, 24c and 38a-38d may be opened to resume flow. When flow resumes, the red blood cell recovery stage may resume or the procedure may be moved on to the next stage. In one alternative, the selected time may be between about 15 seconds and about 45 seconds. In one alternative, the selected time may be about 30 seconds.

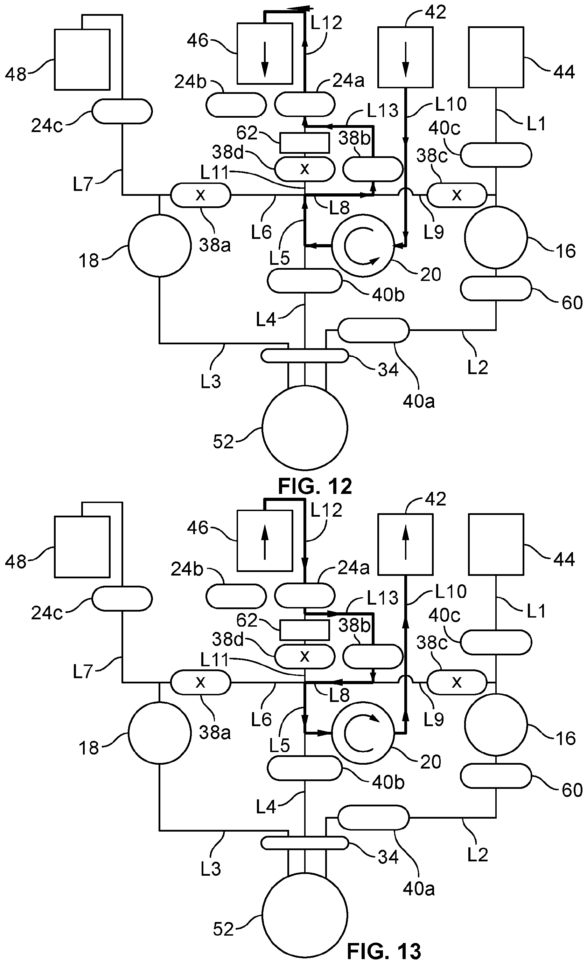

Once the red blood cell recovery stage is complete, the procedure will transition to an "additive solution flush" stage. During the additive solution flush stage, additive solution from the additive solution container 42 is conveyed into the red blood cell collection container 46 until a target amount of additive solution is in the red blood cell collection container 46. The only change in transitioning from the red blood cell recovery stage to the additive solution flush stage involves deactivating the plasma pump to prevent plasma from being removed from the plasma collection container 48 (though it is also possible for the additive pump 20 to operate at a different rate). Thus, if the valve system was arranged to direct flow through the leukoreduction filter 62 at the end of the red blood cell recovery stage (as in Fig. 9), the additive solution flush stage will proceed as shown in Fig. 11. On the other hand, if the valve system was arranged to bypass the leukoreduction filter 62 at the end of the red blood cell recovery stage (as in Fig. 10), the additive solution flush stage will proceed as shown in Fig. 12. If the additive solution is pumped through the leukoreduction filter 62 during the additive solution flush stage (as in Fig. 11), the additive solution flowing through line L11 will flush residual red blood cells in the leukoreduction filter 62 into the red blood cell collection container 46 (in addition to achieving a proper additive solution volume for the red blood cell product).

The additive solution flush stage will continue until a target amount of additive solution has been added to the red blood cell collection container 46. In one exemplary embodiment, the weight of the additive solution container 42 may be monitored by a weight scale, with a particular change in weight corresponding to the target amount of additive solution having been conveyed to the red blood cell collection container 46. Alternatively (or additionally), the weight of the red blood cell collection container 46 may be monitored by a weight scale, with a particular change in weight corresponding to the target amount of additive solution having been conveyed to the red blood cell collection container 46.

When the additive solution flush stage is complete, the system will transition to an "air evacuation" stage, as shown in Fig. 13. During the air evacuation stage, the red blood cell collection container 46 is "burped" to remove all residual air for storage (just as air was removed from the plasma collection container 48 during the red blood cell recovery stage). This is done by reversing the direction of operation of the additive pump 20, closing valve 38d (if not already closed at the end of the additive solution flush stage), and opening valve 38b (if not already open at the end of the additive solution flush stage). The additive pump 20 draws air out of the red blood cell collection container 46, through line L12 and open clamp 24a, into line L13 and through open valve 38b. The air continues through line L8, line L5, and line L10, with the air ending up in the additive solution container 42. While Fig. 13 shows the air being evacuated from the red blood cell collection container 46 to the additive solution container 42, it is within the scope of the present disclosure for all or a portion of the air to be directed to a different location of the fluid flow circuit 12 (e.g., into the processing chamber 52 and/or into the whole blood container 44, if provided).

The air evacuation stage will continue until all of the air is removed from the red blood cell collection container 46, which may be determined (for example) by detecting a change in the weight of the red blood cell collection container 46 (e.g., using a weight scale).

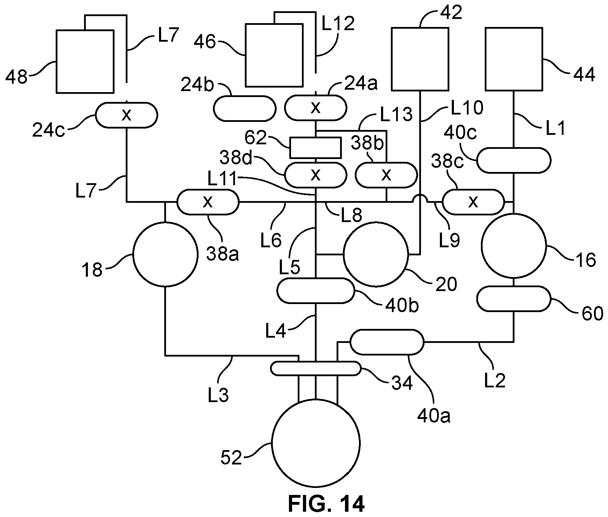

Upon completion of the air evacuation stage, any of a number of post-processing stages may be executed. For example, Fig. 14 shows a "sealing" stage in which all of the clamps and valves are closed and all of the pumps are deactivated. The line L12 connected to the red blood cell collection container 46 and the line L7 connected to the plasma collection container 48 are sealed and optionally severed for storage of the plasma and red blood cell products. If lines L7 and L12 are severed, the plasma collection container 48 and the red blood cell collection container 46 may be stored, while the remainder of the fluid flow circuit 12 is disposed of. Lines L7 and L12 may be sealed (and optionally severed) according to any suitable approach, which may include being sealed by RF sealers incorporated or associated with clamps 24a and 24c, for example. In another embodiment, the fluid flow circuit 12 may be removed from the processing device 10, with lines L7 and L12 being sealed (and optionally severed) using a dedicated sealing device.

Aspects

Aspect 1. A method for separating whole blood, comprising: executing a priming stage in which a pump system and a valve system of a blood processing device are controlled to prime a processing chamber positioned within a centrifuge of the blood processing device; executing a blood separation stage in which the pump system, the valve system, and the centrifuge are controlled to separate blood in the processing chamber into at least two blood components; executing a blood component collection stage in which the pump system and the valve system are controlled to collect at least a portion of one of said at least two blood components; and executing a flow rate stoppage phase to interrupt at least one of the priming, blood separation, and blood component collection stages, the flow rate stoppage phase including: (i) controlling the pumping system and the valve system to prevent fluid flow into and from the processing chamber, (ii) controlling the centrifuge at a selected rate and/or a selected relative centrifugal force; (iii) after a selected time, ending the flow rate stoppage phase; and (iv) resuming the interrupted stage or advancing to a subsequent stage of the method after ending the flow rate stoppage phase.

Aspect 2. The method of Aspect 1, wherein the blood comprises whole blood and the at least two blood components comprise red blood cells and plasma.

Aspect 3. The method of any one of Aspects 1 and 2, wherein the controlling the centrifuge at a selected rate during the flow rate stoppage phase includes spinning at a rate of between 500 and 5500.

Aspect 4. The method of any one of Aspects 1-3, wherein the controlling the centrifuge at a selected rate during the flow rate stoppage phase includes spinning at a rate of about 1500, about 3500 or about 5000.

Aspect 5. The method of any one of Aspects 1-4, wherein the selected time of the flow rate stoppage phase comprises between 15 seconds and 45 seconds.

Aspect 6. The method of any one of Aspects 1-5, wherein the selected time of the flow rate stoppage phase comprises about 30 seconds.

Aspect 7. The method of any one of Aspects 2-6, wherein the blood component collection stage further includes: (i) whole blood being conveyed from a blood source to the processing chamber until a total of one unit of whole blood has been conveyed from the blood source to the processing chamber, and (ii) the centrifuge being controlled to separate the whole blood in the processing chamber into plasma and red blood cells, the separated plasma is conveyed out of the processing chamber and into a plasma collection container, the separated red blood cells are conveyed out of the processing chamber, and an additive solution is conveyed out of an additive solution container of a fluid flow circuit, with the separated red blood cells and the additive solution being combined as a mixture and conveyed into a red blood cell collection container of the fluid flow circuit.

Aspect 8. The method of Aspect 7, further including executing an additive solution flush stage with the pump system and the valve system in which additive solution is conveyed from the additive solution container to the red blood cell collection container until a target amount of additive solution has been conveyed into the red blood cell collection container.

Aspect 9. The method of any one of Aspects 7-8, wherein the fluid flow circuit includes a whole blood container containing one unit of whole blood, and the blood source is the whole blood container.

Aspect 10. The method of any one of Aspects 7-9, wherein said executing blood component collection stage includes measuring a weight of the whole blood container, and ending the blood component collection stage based at least in part on the weight of the whole blood container.

Aspect 11. The method of Aspect 10, wherein the blood source is a living donor.

Aspect 12. The method of any one of Aspects 7-11, wherein said executing the blood component collection stage further includes measuring hydrostatic pressure of the whole blood container, and ending the blood component collection stage based at least in part on the hydrostatic pressure of the whole blood container.

Aspect 13. The method of any one of Aspects 7-12, wherein said executing the blood component collection stage includes conveying the mixture through a leukoreduction filter before being conveyed into the red blood cell collection container during at least a portion of the blood component collection stage.

Aspect 14. The method of any one of Aspects 2-13, wherein the priming stage comprises conveying whole blood from the blood source to a processing chamber to remove air from the processing chamber.

Aspect 15. The method of any one of Aspect 1-14, wherein said executing the priming stage includes monitoring fluid exiting the processing chamber, and ending the priming stage when a non-air fluid is detected exiting the processing chamber.

Aspect 16. The method of any one of Aspect 2-15, wherein the blood separation stage comprises conveying the separated plasma and red blood cells out of the processing chamber and recombing the plasma and red blood cells as recombined whole blood, and conveying the recombined whole blood into the processing chamber.

Aspect 17. The method of any one of Aspects 1-16, further including executing an air flush stage with the pump system and the valve system in which air is conveyed into the processing chamber to convey separated at least one of the separated blood components out of the processing chamber.

Aspect 18. The method of any one of Aspects 1-17, wherein the pump system comprises a plurality of pumps, and executing a flow rate stoppage phase comprises stopping one or more of the plurality of pumps.

Aspect 19. The method of any one of Aspects 1-18, wherein the valve system comprises a plurality of clamps, and executing a flow rate stoppage phase comprises closing one or more of the plurality of clamps.

Aspect 20. The method of any one of Aspects 1-19, wherein in the selected relative centrifugal force is between about 10Gs and about 1450Gs.

Aspect 21. The method of any one of Aspects 1-20, wherein the selected relative centrifugal force is about 100Gs or about 1140Gs.

Aspect 22. A blood processing device, comprising: a pump system; a valve system; a centrifuge; and a controller, wherein the controller is configured to execute a blood separation procedure including executing a priming stage in which the pump system and the valve system are controlled to prime a processing chamber positioned within the centrifuge; executing a blood separation stage in which the pump system, the valve system, and the centrifuge are controlled to separate blood in the processing chamber into at least two blood components; executing a blood component collection stage in which the pump system and the valve system are controlled to collect at least a portion of one of said at least two blood components; and executing a flow rate stoppage phase to interrupt at least one of the priming, blood separation, and blood component collection stages, the flow rate stoppage phase including: (i) controlling the pumping system and the valve system to prevent fluid flow into and from the processing chamber, (ii) controlling the centrifuge at a selected rate; (iii) after a selected time, ending the flow rate stoppage phase; and (iv) resuming the interrupted stage or advancing to a subsequent stage of the blood separation procedure after ending the flow rate stoppage phase.

Aspect 23. The blood processing device of Aspect 22, wherein the blood comprises whole blood and the at least two blood components comprise red blood cells and plasma.

Aspect 24. The blood processing device of any one of Aspects 22 and 23, wherein the controlling the centrifuge at a selected rate during the flow rate stoppage phase includes spinning at a rate of between 500 and 5500.

Aspect 25. The blood processing device of any one of Aspects 23-24, wherein the controlling the centrifuge at a selected rate during the flow rate stoppage phase includes spinning at a rate of about 1500, about 3500 or about 5000.

Aspect 26. The blood processing device of any one of Aspects 22-25, wherein the selected time of the flow rate stoppage phase comprises between 15 seconds and 45 seconds.

Aspect 27. The blood processing device of any one of Aspects 22-26, wherein the selected time of the flow rate stoppage phase comprises about 30 seconds.

Aspect 28. The blood processing device of any one of Aspects 23-27, wherein the blood component collection stage further includes: (i) whole blood being conveyed from a blood source to the processing chamber until a total of one unit of whole blood has been conveyed from the blood source to the processing chamber, and (ii) the centrifuge being controlled to separate the whole blood in the processing chamber into plasma and red blood cells, the separated plasma is conveyed out of the processing chamber and into a plasma collection container, the separated red blood cells are conveyed out of the processing chamber, and an additive solution is conveyed out of an additive solution container of a fluid flow circuit, with the separated red blood cells and the additive solution being combined as a mixture and conveyed into a red blood cell collection container of the fluid flow circuit.

Aspect 29. The blood processing device of Aspect 28, further including executing an additive solution flush stage with the pump system and the valve system in which additive solution is conveyed from the additive solution container to the red blood cell collection container until a target amount of additive solution has been conveyed into the red blood cell collection container.

Aspect 30. The blood processing device of any one of Aspects 28-29, wherein the fluid flow circuit includes a whole blood container containing one unit of whole blood, and the blood source is the whole blood container.

Aspect 31. The blood processing device of any one of Aspects 28-30, wherein said executing the blood component collection stage includes measuring a weight of the whole blood container, and ending the blood component collection stage based at least in part on the weight of the whole blood container.

Aspect 32. The blood processing device of Aspect 31, wherein the blood source is a living donor.

Aspect 33. The blood processing device of any one of Aspects 28-32, wherein said executing the blood component collection stage further includes measuring hydrostatic pressure of the whole blood container, and ending the blood component collection stage based at least in part on the hydrostatic pressure of the whole blood container.

Aspect 34. The blood processing device of any one of Aspects 28-32, wherein said executing the blood component collection stage includes conveying the mixture through a leukoreduction filter before being conveyed into the red blood cell collection container during at least a portion of the blood component collection stage.

Aspect 35. The blood processing device of any one of Aspects 23-34, wherein the priming stage comprises conveying whole blood from the blood source to a processing chamber to remove air from the processing chamber.

Aspect 36. The blood processing device of any one of Aspect 22-35, wherein said executing the priming stage includes monitoring fluid exiting the processing chamber, and ending the priming stage when a non-air fluid is detected exiting the processing chamber.

Aspect 37. The blood processing device of any one of Aspect 23-36, wherein the blood separation stage comprises conveying the separated plasma and red blood cells out of the processing chamber and recombing the plasma and red blood cells as recombined whole blood, and conveying the recombined whole blood into the processing chamber.

Aspect 38. The blood processing device of any one of Aspects 22-37, further including executing an air flush stage with the pump system and the valve system in which air is conveyed into the processing chamber to convey separated at least one of the separated blood components out of the processing chamber.

Aspect 39. The blood processing device of any one of Aspects 22-8, wherein the pump system comprises a plurality of pumps, and executing a flow rate stoppage phase comprises stopping one or more of the plurality of pumps.

Aspect 40. The blood processing device of any one of Aspects 22-39, wherein the valve system comprises a plurality of clamps, and executing a flow rate stoppage phase comprises closing one or more of the plurality of clamps.

Aspect 41. The blood processing device of any one of Aspects22-40, wherein in the selected relative centrifugal force is between about 10Gs and about 1450Gs.

Aspect 42. The blood processing device of any one of Aspect 22-41, wherein the selected relative centrifugal force is about 100Gs or about 1140Gs.

**Claims**

1. A blood processing device, comprising:

   a pump system;
   a valve system;
   a centrifuge; and
   a controller, wherein the controller is configured to execute a blood separation procedure including

      executing a priming stage in which the pump system and the valve system are controlled to prime a processing chamber positioned within the centrifuge;
      executing a blood separation stage in which the pump system, the valve system, and the centrifuge are controlled to separate blood in the processing chamber into at least two blood components;
      executing a blood component collection stage in which the pump system and the valve system are controlled to collect at least a portion of one of said at least two blood components; and
      executing a flow rate stoppage phase to interrupt at least one of the priming, blood separation, and blood component collection stages, the flow rate stoppage phase including:

         (i) controlling the pumping system and the valve system to prevent fluid flow into and from the processing chamber,
         (ii) controlling the centrifuge at a selected rate and/or a selected relative centrifugal force;
         (iii) after a selected time, ending the flow rate stoppage phase; and
         (iv) resuming the interrupted stage or advancing to a subsequent stage of the blood separation procedure after ending the flow rate stoppage phase.

2. The blood processing device of claim 1, wherein the blood comprises whole blood and the at least two blood components comprise red blood cells and plasma.

3. The blood processing device of any one of claims 1 and 2, wherein the controlling the centrifuge at a selected rate during the flow rate stoppage phase includes spinning at a rate of between 500 and 5500.

4. The blood processing device of any one of claims 1-3, wherein the controlling the centrifuge at a selected rate during the flow rate stoppage phase includes spinning at a rate of about 1500, about 3500 or about 5000.

5. The blood processing device of any one of claims 1-4, wherein the selected time of the flow rate stoppage phase comprises between 15 seconds and 45 seconds, and preferably about 30 seconds.

6. The blood processing device of any one of claims 1-5, wherein the blood component collection stage further includes:

   (i) whole blood being conveyed from a blood source to the processing chamber until a total of one unit of whole blood has been conveyed from the blood source to the processing chamber, and
   (ii) the centrifuge being controlled to separate the whole blood in the processing chamber into plasma and red blood cells, the separated plasma is conveyed out of the processing chamber and into a plasma collection container, the separated red blood cells are conveyed out of the processing chamber, and an additive solution is conveyed out of an additive solution container of a fluid flow circuit, with the separated red blood cells and the additive solution being combined as a mixture and conveyed into a red blood cell collection container of the fluid flow circuit.

7. The blood processing device of claim 6, further including executing an additive solution flush stage with the pump system and the valve system in which additive solution is conveyed from the additive solution container to the red blood cell collection container until a target amount of additive solution has been conveyed into the red blood cell collection container.

8. The blood processing device of any one of claims 6-7, wherein

the fluid flow circuit includes a whole blood container containing one unit of whole blood, and
the blood source is the whole blood container.

9. The blood processing device of any one of claims 6-8, wherein said executing the blood component collection stage includes

measuring a weight of the whole blood container, and
ending the blood component collection stage based at least in part on the weight of the whole blood container.

10. The blood processing device of any one of claims 6-9, wherein said executing the blood component collection stage further includes

measuring hydrostatic pressure of the whole blood container, and
ending the blood component collection stage based at least in part on the hydrostatic pressure of the whole blood container.

11. The blood processing device of any one of claims 6-10, wherein said executing the blood component collection stage includes conveying the mixture through a leukoreduction filter before being conveyed into the red blood cell collection container during at least a portion of the blood component collection stage.

12. The blood processing device of any one of claims 2-11, wherein the priming stage comprises conveying whole blood from the blood source to a processing chamber to remove air from the processing chamber.

13. The blood processing device of any one of claim 2-12, wherein the blood separation stage comprises conveying the separated plasma and red blood cells out of the processing chamber and recombing the plasma and red blood cells as recombined whole blood, and conveying the recombined whole blood into the processing chamber.

14. The blood processing device of any one of claims 1-13, further including executing an air flush stage with the pump system and the valve system in which air is conveyed into the processing chamber to convey separated at least one of the separated blood components out of the processing chamber.

15. The blood processing device of any one of claims 1-14, wherein the pump system comprises a plurality of pumps and the valve system comprises a plurality of clamps, and executing a flow rate stoppage phase comprises stopping one or more of the plurality of pumps and/or closing one or more of the plurality of clamps.

**FIG. 1**

**FIG. 2**

FIG. 3

FIG. 4

FIG. 5A

70

Commence/continue/resume a stage of
the method (Prime, Collection, etc.)
Pumps: ON
Centrifuge: ON

no

Has pre-
determined
time past?

72

yes

74

Commence/continue the flow rate
stoppage phase (stoppage phase):
Pumps: OFF
Optionally, valves: CLOSED
Centrifuge: ON
Set centrifuge to pre-determined RPM

no

Has pre-
selected
time past?

76

yes

no          78

Has an END
condition to end
the need of the
stoppage phase
been met?

yes

Commence/resume a stage of the
procedure (Prime, Collection, etc.)
Pumps: ON
Centrifuge: ON

80

**FIG. 5B**

82

Commence/continue/resume a stage of
the procedure (Prime, Collection, etc.)
Pumps: ON
Centrifuge: ON

no

Has a flow rate
stoppage condition
been met?

84

yes

86

Commence the flow rate stoppage phase:
Pumps: OFF
Optionally, valves: CLOSED
Centrifuge: ON
Set centrifuge to pre-determined RPM

no

Has pre-
selected
time past?

yes

88

# FIG. 5C

FIG. 6

FIG. 7

**FIG. 8**

**FIG. 9**

FIG. 10

FIG. 11

FIG. 12

FIG. 13

**FIG. 14**

Europäisches
Patentamt

European
Patent Office

Office européen
des brevets

# EUROPEAN SEARCH REPORT

Application Number

EP 23 15 1656

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 0 214 803 A2 (FISONS PLC [GB]) 18 March 1987 (1987-03-18) | 1-5,9,15 | INV. A61M1/36 |
| Y | * abstract; figure 1 * <br> * page 20, line 21 - page 23, line 22 * | 6-8, 10-14 | |
| Y | WO 2021/194824 A1 (FENWAL INC [US]) 30 September 2021 (2021-09-30) * abstract; figures * * page 3, line 20 - page 8, line 1 * * page 15, lines 25-32 * * page 21, line 29 - page 22, line 15 * * page 22, line 16 * | 6-8, 10-14 | |
| A | US 2002/177799 A1 (RIVERA JOHN [US] ET AL) 28 November 2002 (2002-11-28) * abstract; claim 6; figures * * paragraphs [0008] - [0010] * | 1-15 | |
| A | US 2008/035585 A1 (ANTWILER GLEN DELBERT [US]) 14 February 2008 (2008-02-14) * abstract; figures * * paragraphs [0045] - [0053] * | 1-15 | TECHNICAL FIELDS SEARCHED (IPC) <br><br> A61M |

The present search report has been drawn up for all claims

| Place of search | Date of completion of the search | Examiner |
|---|---|---|
| The Hague | 5 June 2023 | Kaden, Malte |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 15 1656

This annex lists the patent family members relating to the patent documents cited in the above-mentioned European search report.
The members are as contained in the European Patent Office EDP file on
The European Patent Office is in no way liable for these particulars which are merely given for the purpose of information.

05-06-2023

| Patent document cited in search report | | Publication date | Patent family member(s) | | | Publication date |
|---|---|---|---|---|---|---|
| EP 0214803 | A2 | 18-03-1987 | AU | 6213786 | A | 05-03-1987 |
| | | | DK | 410186 | A | 04-03-1987 |
| | | | EP | 0214803 | A2 | 18-03-1987 |
| | | | FR | 2586565 | A1 | 06-03-1987 |
| | | | GB | 2183178 | A | 03-06-1987 |
| | | | JP | S62106769 | A | 18-05-1987 |
| | | | PT | 83289 | A | 06-05-1987 |
| WO 2021194824 | A1 | 30-09-2021 | EP | 4126096 | A1 | 08-02-2023 |
| | | | JP | 2023518823 | A | 08-05-2023 |
| | | | WO | 2021194824 | A1 | 30-09-2021 |
| US 2002177799 | A1 | 28-11-2002 | AU | 2116597 | A | 25-08-1998 |
| | | | EP | 1015044 | A1 | 05-07-2000 |
| | | | EP | 1972353 | A1 | 24-09-2008 |
| | | | US | 5964724 | A | 12-10-1999 |
| | | | US | 6475175 | B1 | 05-11-2002 |
| | | | US | 2002177799 | A1 | 28-11-2002 |
| | | | WO | 9833534 | A1 | 06-08-1998 |
| US 2008035585 | A1 | 14-02-2008 | US | 2008035585 | A1 | 14-02-2008 |
| | | | WO | 2008021623 | A1 | 21-02-2008 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 8075468 B
- US 4157723 A
- US 4753697 A
- US 5158630 A
- US 5156701 A
- US 10307582 B
- US 10040247 B
- US 9440396 B
- US 20190201916
- US 6849039 B
- US 6899666 B
- US 20180078582